(19) Europäisches Patentamt
European Patent Office
Office européen des brevets

(11) **EP 3 357 910 A1**

(12) **EUROPEAN PATENT APPLICATION**

(43) Date of publication:
**08.08.2018 Bulletin 2018/32**

(21) Application number: **17154441.4**

(22) Date of filing: **02.02.2017**

(51) Int Cl.:
***C07D 213/73*** *(2006.01)* ***A61K 51/04*** *(2006.01)*
***C07D 213/74*** *(2006.01)* ***C07D 213/82*** *(2006.01)*
***C07D 213/84*** *(2006.01)* ***C07D 217/14*** *(2006.01)*
***C07D 405/04*** *(2006.01)* ***C07D 241/12*** *(2006.01)*
***C07D 241/16*** *(2006.01)* ***C07D 241/18*** *(2006.01)*
***C07D 241/20*** *(2006.01)* ***C07D 241/24*** *(2006.01)*
***C07D 213/57*** *(2006.01)* ***C07D 213/61*** *(2006.01)*
***C07D 213/65*** *(2006.01)* ***C07D 213/70*** *(2006.01)*
***A61K 31/44*** *(2006.01)* ***A61K 31/47*** *(2006.01)*
***A61K 31/4965*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Julius-Maximilians-Universität Würzburg**
**97070 Würzburg (DE)**

(72) Inventors:
- **Schirbel, Andreas**
  **45888 Gelsenkirchen (DE)**
- **Heinze, Britta**
  **97222 Rimpar (DE)**
- **Hahner, Stefanie**
  **97074 Würzburg (DE)**

(74) Representative: **Dr. Gassner & Partner mbB**
**Marie-Curie-Str. 1**
**91052 Erlangen (DE)**

(54) **COMPOUND FOR IN VIVO DIAGNOSIS OF A DYSFUNCTION OF ADRENAL GLANDS**

(57) The invention concerns a compound having formula (I):

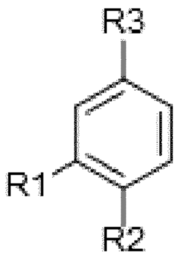 (I)

,

wherein R1 represents a radioactive isotope of an element which radioactive isotope is detectable by positron emission tomography (PET) or wherein R1 represents any nonradioactive isotope of said element, R2 represents -CN or -$CF_3$, wherein R3 represents

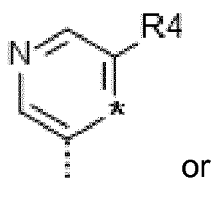 or 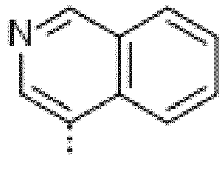 ,

wherein * represents C or N and R4 represents one of the moieties of a group consisting of alkyl, alkyl ether, alkyl nitrile, alkyl alcohol, alkyl halide, O-alkyl, S-alkyl, halogen, nitrile, trifluoromethyl, amino, aminoalkyl, aminodialkyl, phenyl, substituted phenyl, heterocyclic aromatic or a substituted heterocyclic aromatic, dioxolanyl, methyldioxolanyl, and dioxanyl.

EP 3 357 910 A1

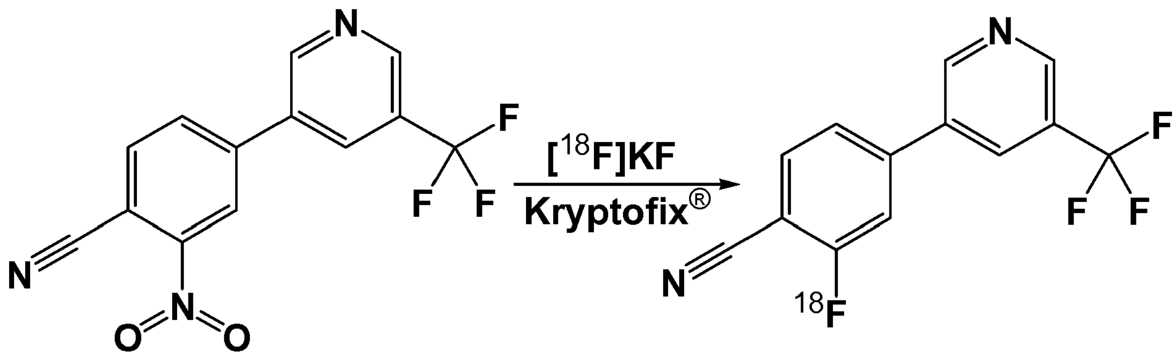


Fig. 4

## Description

The invention concerns an alternative compound for *in vivo* diagnosis of a dysfunction of adrenal glands, in particular primary hyperaldosteronism, the use of that compound in an *in vivo* method of diagnosis and a method for synthesizing that compound.

Adrenal vein sampling is the standard method to distinguish unilateral from bilateral forms of primary aldosteronism. It is an invasive and technically difficult procedure. $^{11}$C-metomidate (MTO)-positron emission tomography was reported as a potential replacement for adrenal vein sampling. However, MTO has low selectivity for CYP11 B2 (aldosterone synthase) over CYP11 B1 (11ß-hydroxylase).

WO 2011/151411 A1 discloses a functional positron emission tomography (PET) imaging method for differentiation between bilateral hyperplasia and unilateral adenoma comprising (1) introducing a radioactively labeled CYP11 B2 inhibitor which binds selectively to CYP11 B2 relative to CYP11 B1 into a mammal with adrenal glands and (2) conducting PET in the region of the adrenal glands to obtain a functional PET image of the adrenal glands. Also disclosed are radioactive tracer compounds suitable for use in this method, precursors for making the same, and a process for making the radioactive tracer compounds capable of being conducted as a rapid one-pot reaction.

From Abe, T. et al., J. Clin. Endocrinol. Metab., March 2016, 101(3): 1008-15, a novel CYP11 B2-specific imaging agent for detection of unilateral subtypes of primary aldosteronism is known. Selectivity of $^{18}$F-CDP2230, a new imaging agent, for CYP11 B2 over CYP11 B1 has been shown. The $IC_{50}$ of CDP2230 for the enzymatic activities of CYP11 B2 and CYP11 B1 was determined using cells with stable expression of either enzyme. *In vitro* autoradiography of human adrenal sections with aldosterone-producing adenomas was performed to confirm the specific binding ability of $^{18}$F-CDP2230 to CYP11 B2-expressing regions.

Furthermore, positron emission tomography and magnetic resonance imaging were performed to evaluate the biodistribution of $^{18}$F-CDP2230 in rats. Although CDP2230 showed a significantly lower affinity for CYP11B2 and CYP11B1 than did MTO analogues, its selectivity for CYP11B2 over CYP11B1 was higher than that of MTO analogues. *In vitro* autoradiography revealed that the binding of $^{18}$F-CDP2230 to CYP11B2-expressing regions in the adrenal gland was more specific than that of $^{123}$I-IMTO. Moreover, the biodistribution study showed that $^{18}$F-CDP2230 accumulated in adrenal glands with low background uptake. A high selectivity of $^{18}$F-CDP2230 for CYP11B2 over CYP11B1 with a favorable biodistribution for imaging CYP11B2 has been shown.

The problem to be solved by the present invention is to provide a further compound suitable for an *in vivo* method of diagnosis, in particular a functional diagnosis of a dysfunction of adrenal glands. Furthermore, a problem to be solved by the present invention is to provide said compound for use in an *in vivo* method of diagnosis and a method for synthesizing said compound.

The problems are solved by the features of claims 1, 7 and 12. Embodiments of these features are disclosed in claims 2 to 6, 8 to 11 and 13 to 15.

According to the invention a compound having formula (I) is provided:

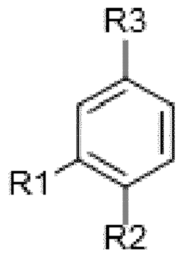

(I)

.

R1 represents a radioactive isotope of an element which radioactive isotope is detectable by positron emission tomography (PET) or any nonradioactive isotope of said element. This means that the element is in any case an element having at least one isotope that is detectable by PET. R2 represents -CN or $-CF_3$. R3 represents

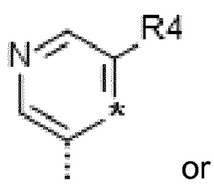 or 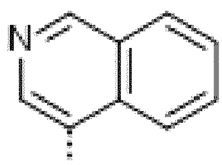 ,

wherein * represents CH or N and R4 represents one of the moieties of a group consisting of alkyl, alkyl ether, alkyl nitrile, alkyl alcohol, alkyl halide, O-alkyl, S-alkyl, halogen, nitrile, trifluoromethyl, amino, aminoalkyl, aminodialkyl, phenyl, substituted phenyl, heterocyclic aromatic, substituted heterocyclic aromatic, dioxolanyl, methyldioxolanyl, and dioxanyl.

A radioactive isotope detectable by PET may be, e. g., $^{11}C$, $^{13}N$, $^{15}O$, $^{18}F$, $^{68}Ga$, or $^{82}Rb$. In particular the radioactive isotope may be $^{18}F$.

The inventors found that the compound according to the invention binds in particular to CYP11B2 and also to CYP11B1. Many embodiments of the compound according to the invention have a high selectivity for CYP11B2 vis-à-vis CYP11B1. Thus, they allow for a differential diagnosis of unilateral and bilateral forms of primary aldosteronism by administration to a mammal followed by PET imaging on basis of different distribution in the adrenal glands in both forms of primary aldosteronism. If R1 is the nonradioactive isotope, the compound can be used in combination with a PET tracer compound that is not specific for CYP11B1 and CYP11B2 such as $^{125}$I-Iodometomidate (IMTO). Such a nonspecific tracer compound may then specifically be displaced by the nonradioactive compound according to the invention from CYP11B2 thus allowing a differential diagnosis of unilateral and bilateral forms of primary aldosteronism.

Interestingly, the inventors also found that * can be N though the pyridine ring in which * is C is considered as pharmacophore. In a pharmacophore alterations are usually not possible without losing effect or binding affinity.

Furthermore, the inventors found that the radioactive labeling in the compound according to the invention is chemically and metabolically very stable. It is assumed that this is caused by the direct binding of the radioactive isotope to the phenyl ring.

A big advantage of the compound according to the invention is that the molecule(s) to which the radioactive or nonradioactive isotope has to be bound to obtain a compound according to the invention do not contain functional groups that may interfere with a coupling reaction. Therefore, it is not required to use one or more protective group(s) to perform an efficient labeling reaction. As a consequence, the compound according to the invention can be synthesized in a simple and effective one-pot reaction if suitable precursors are provided. This allows a high radiochemical yield within a short reaction time and an easy automatization of radiosynthesis.

In an embodiment R4 represents one of the following moieties: halogen, $-OCH_3$, $-CH_2OH$, $-CH_3$, $-N(CH_3)_2$, $-CH_2(CN)CH_3$, phenyl, substituted phenyl, heterocyclic aromatic and substituted heterocyclic aromatic. The compound according to the invention may be

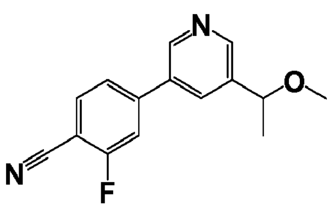 , 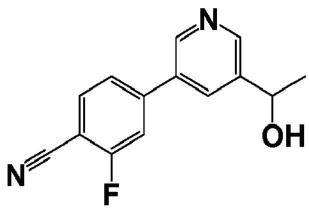 , 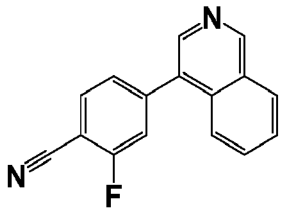 ,

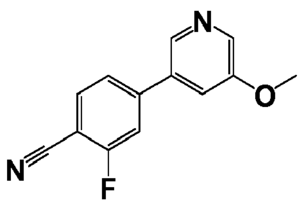 , 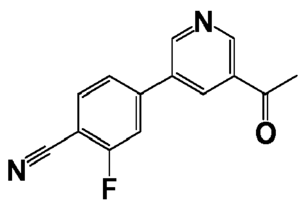 , 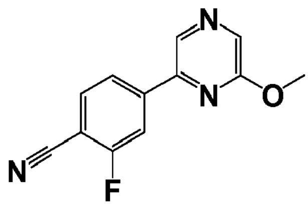 ,

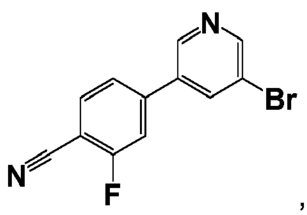 , 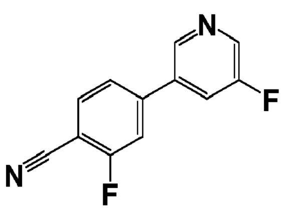 , 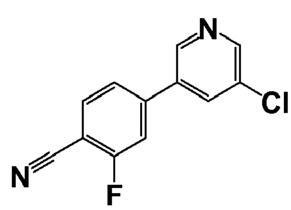 ,

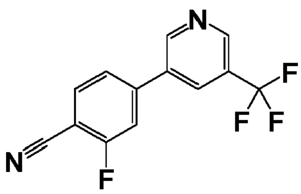 , 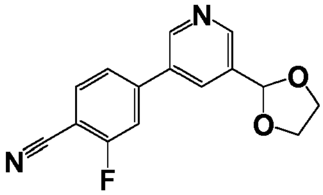 , 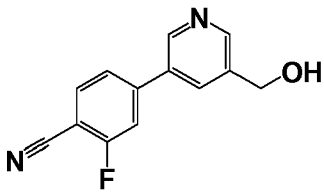 ,

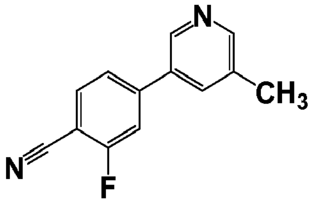 , 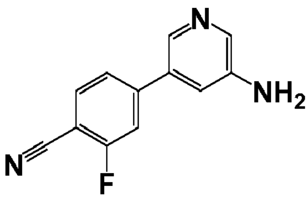 , 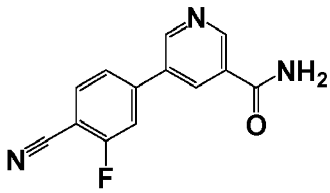 ,

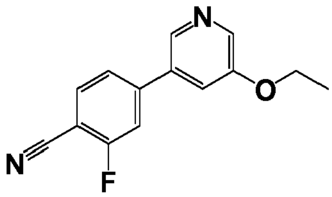 , 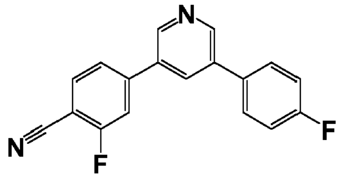 , 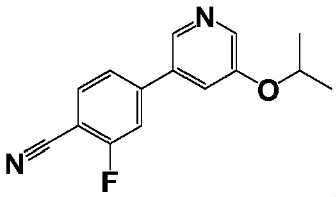 ,

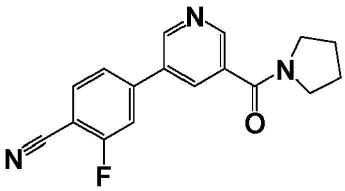 , 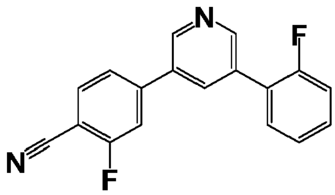 , 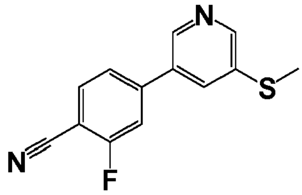 ,

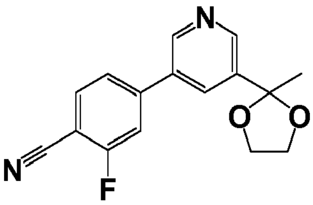 , 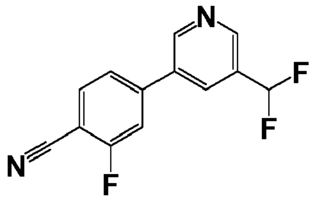 , 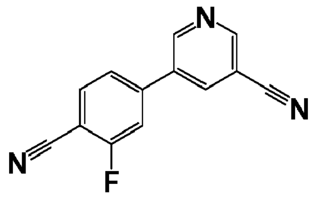 ,

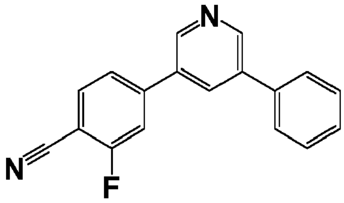 , 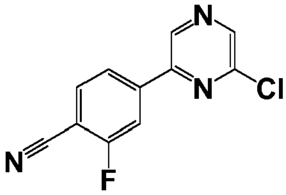 , 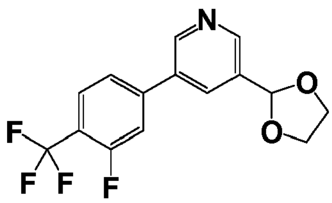 ,

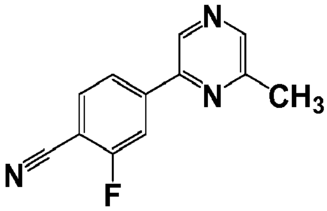 , 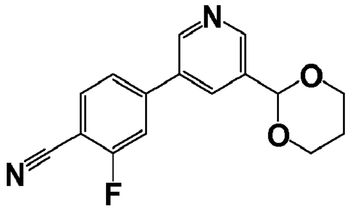 , 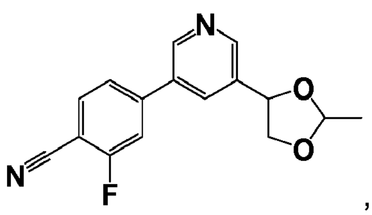 ,

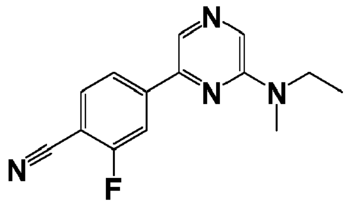, 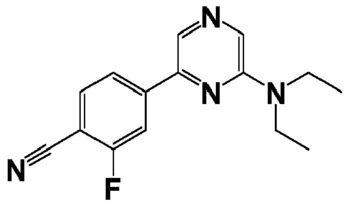, 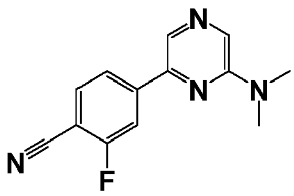,

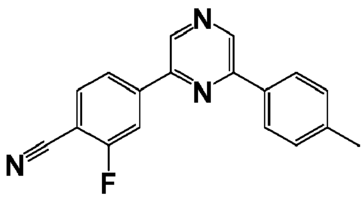, 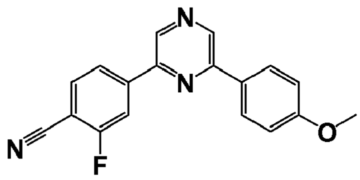, 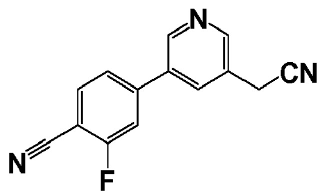,

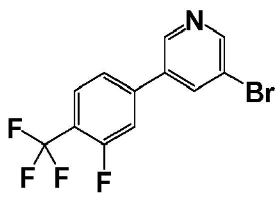, 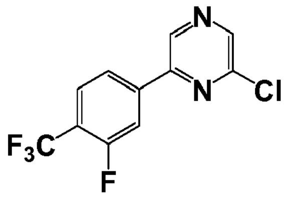, 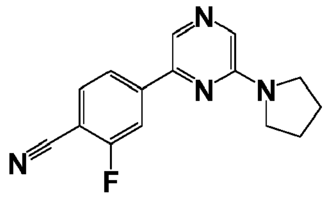,

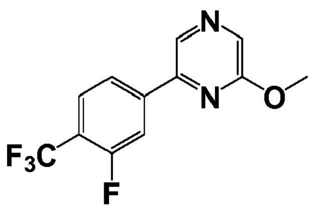, 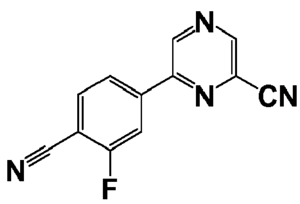, 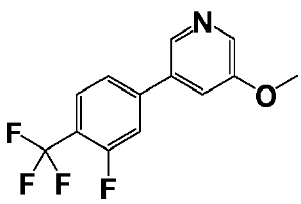,

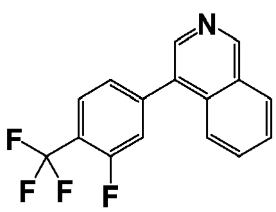, 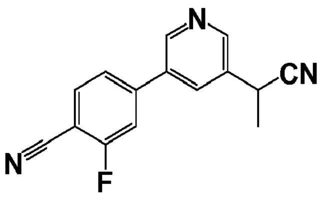, 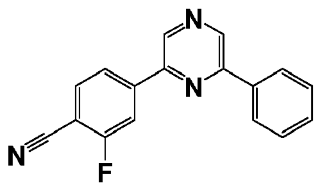,

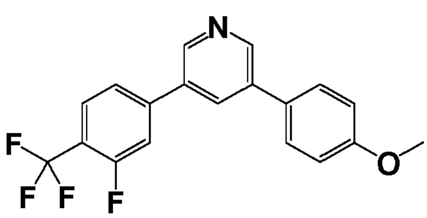, 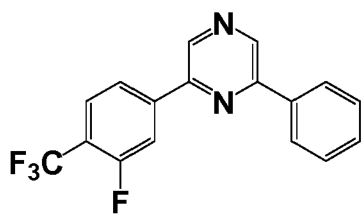,

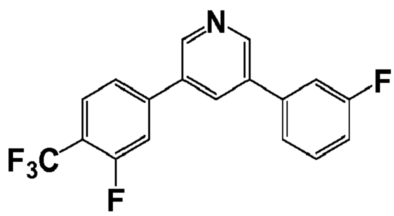, or 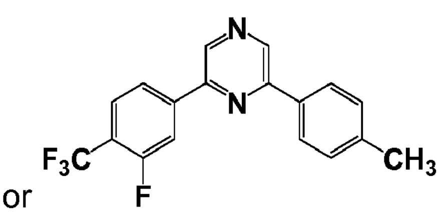.

In anyone of these compounds the F directly bound to the phenyl ring can be $^{18}F$.

In an embodiment of the invention a specificity factor of the compound of the invention calculated as a relation

of an $IC_{50}$ value of an enzymatic reaction of human CYP11B1 to an $IC_{50}$ value of an enzymatic reaction of human CYP11B2 is at least 10, in particular at least 15, in particular at least 20, in particular at least 25, in particular at least 30, in particular at least 35, in particular at least 40, in particular at least 45, in particular at least 50, in particular at least 55, in particular at least 60, in particular at least 65, in particular at least 70, in particular at least 75, in particular at least 80, in particular at least 85. $IC_{50}$ is the half maximal inhibitory concentration. It is a measure of the effectiveness of a substance in inhibiting a specific biological or biochemical function. The enzymatic reaction may be determined in cell culture medium of NCI-H295 cells or of murine Y1-cells stably transfected with human CYP11B1 gene (Y1-Cyp11B1 cells) and in cell culture medium of other murine Y1-cells stably transfected with human CYP11B2 gene (Y1-Cyp11B2 cells) as described below.

The invention further concerns the compound according to the invention for use in an *in vivo* method of diagnosis. The diagnosis may be a functional diagnosis of a dysfunction of adrenal glands. In an embodiment the dysfunction of adrenal glands is a unilateral form or a bilateral form of primary aldosteronism. The method of diagnosis may be positron emission tomography (PET). In an embodiment the method of diagnosis comprises introducing any compound according to the invention into a mammal with adrenal glands and conducting PET of the adrenal glands or in the region of the adrenal glands to obtain a functional image of the adrenal glands. In this case R1 represents the radioactive isotope.

The invention further concerns a method for synthesizing the compound according to the invention, wherein a first precursor according to formula (II)

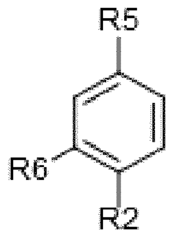

(II)

and a second precursor according to formula (III)

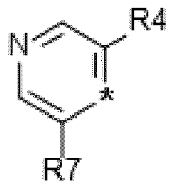

(III)

or a second precursor according to formula (IV)

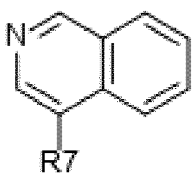

(IV)

are coupled with each other by a coupling reaction catalyzed by a catalyst, wherein R5 and R7 are moieties enabling the coupling reaction for coupling the first precursor with the second precursor, wherein R6 is R1 or a nitro group that is replaced by F or $^{18}F$ by a reaction with potassium fluoride or [$^{18}F$] potassium fluoride in the presence of a cryptand prior or after the coupling reaction. The coupling reaction may be a Suzuki reaction. In an embodiment one of R5 and R7 is $-B(OH)_2$ and the other of R5 and R7 is a halogen moiety, in particular a bromine moiety. The catalyst may be tetrakis(triphenylphosphine)palladium(0). The cryptand may be 4,7,13,16,21,24-Hexaoxa-1,10-diazabicyclo[8.8.8]hexacosane ($C_{18}H_{36}N_2O_6$). This cryptand can be purchased from Merck KGaA, Frankfurter Straße 250, 64293 Darmstadt, Germany under the commercial name Kryptofix® 222. The function of the cryptand is the complexation of potassium ions thus facilitating the reaction of the fluoride ions with the first precursor according to formula (II) or with the molecule resulting from the coupling of the first precursor with the second precursor.

Embodiments of the invention:

Fig. 1 shows a reaction scheme of a reaction for synthesis of a compound according to the invention.

Fig. 2 shows a reaction scheme of a reaction for synthesis of a precursor.

Fig. 3 shows a reaction scheme of a reaction of the precursor for synthesis of a further precursor.

Fig. 4 shows a reaction scheme of a reaction of the further precursor for synthesis of a compound according to the invention.

Fig. 5 shows the results of binding assays performed at cryosections of human tissue.

Fig. 6 shows immunohistological stainings of adrenal gland tissues.

Fig. 7 to 9 show effects of several substances and inhibitors on ex vivo aldosterone production.

Fig. 10 shows the effect of several substances and one inhibitor on *in vivo* aldosterone production.

1. Synthesis of 3-(4-Cyano-3-fluorophenyl)-5-(trifluoromethyl)pyridine

A suspension of 412 mg (2.5 mmol) 4-cyano-3-fluorophenylboronic acid, 565 mg (2.5 mmol) 3-bromo-5-(trifluoromethyl)pyridine, 760 mg (2.75 mmol) silver carbonate and 174 mg (0.15 mmol) tetrakis triphenylphosphine palladium(0) in 30 mL benzene was refluxed for 2 days. After cooling to room temperature the mixture was poured into 100 mL water, extracted three times each with 50 mL methyl-*tert*-butylether, and dried over sodium sulfate. After stripping the solvent the crude product was purified by column chromatography using $CH_2Cl_2$/MeOH 98/2 (vol/vol) as eluent. The yield was 35 mg (0.15 mmol, 6.2%). A reaction scheme of this reaction is shown in Fig. 1.

The purified product showed the following characteristics:

Appearance: Yellow viscous oil

Result of thin-layer chromatography (silica gel):

$R_f$ 3-bromo-5-(trifluoromethyl)pyridine ($CH_2Cl_2$/$CH_3OH$ 98/2 (vol/vol)) = 0.85
$R_f$ 3-(4-cyano-3-fluorophenyl)-5-(trifluoromethyl)pyridine ($CH_2Cl_2$/MeOH 98/2 (vol/vol)) = 0.50

$^1$H-NMR ($CDCl_3$): $\delta$ = 9.05 (bs, 1 H), 8.98 (bs, 1 H), 8.12 (s, 1 H), 7.82 (t, 1 H), 7.52 (dt, 2H)

2. Synthesis of 3-(4-Cyano-3-nitrophenyl)-5-(trifluoromethyl)pyridine in a 2-Step procedure

Step 1: Synthesis of 4-Bromo-2-nitrobenzonitrile

A suspension of 10.0 g (35.6 mmol) 1,4-dibromo-2-nitrobenzene and 3.35 g (37.4 mmol) copper(I)cyanide in 40 mL dimethylacetamide (DMMA) was heated under stirring for 5 h at 100 °C. After cooling to room temperature the mixture was poured into 500 mL ethyl acetate and filtered. The filtrate was washed three times each with 400 mL water and dried over sodium sulfate. After stripping the solvent the crude product was purified by column chromatography (heptane/EtOAc 80/20 (vol/vol)). The yield was 5.02 g (22.1 mmol, 62.1 %). A reaction scheme of this reaction is shown in Fig. 2.

The purified product showed the following characteristics:

Appearance: Yellow crystals

Melting point: 100-101.5 °C

Result of thin-layer chromatography (silica gel):

$R_f$ 1,4-dibromo-2-nitrobenzene (heptane/EtOAc 80/20 (vol/vol))= 0.60
$R_f$ 4-bromo-2-nitrobenzonitrile (heptane/EtOAc 80/20 (vol/vol))= 0.30

$^1$H-NMR ($CDCl_3$): $\delta$ = 8.49 (d, 1 H), 7.97 (dd, 1 H), 7.79 (d, 1 H)

Step 2: Synthesis of 3-(4-Cyano-3-nitrophenyl)-5-(trifluoromethyl)pyridine

250 mg (1.31 mmol) 4-cyano-3-fluorophenylboronic acid, 329 mg (1.45 mmol) 4-bromo-2-nitrobenzonitrile, 3.1 mL of a 2 M solution of sodium carbonate in water and 89 mg (0.073 mmol) tetrakis triphenylphosphine palladium(0) were suspended in 9 mL dimethoxyethane and refluxed over night. After cooling to room temperature the mixture was poured into 50 mL water, extracted three times each with 25 mL methyl-*tert*-butylether, and dried over sodium sulfate. After stripping the solvent the crude product was purified by column chromatography ($CH_2Cl_2$/MeOH 98/2 (vol/vol)). The yield was 226.3 mg (0.77 mmol, 58.9%). A reaction scheme of this reaction is shown in Fig. 3.

The purified product showed the following characteristics:

Appearance: Yellow oil

Thin-layer chromatography (silica gel):

$R_f$ 4-bromo-2-nitrobenzonitrile ($CH_2Cl_2/CH_3OH$ 98/2(vol/vol)) = 0.80
$R_f$ 3-(4-Cyano-3-nitrophenyl)-5-(trifluoromethyl)pyridine ($CH_2Cl_2$/MeOH 98/2(vol/vol)) = 0.40

$^1$H-NMR ($CDCl_3$): $\delta$ = 9.13 (d, 1 H), 9.04 (d, 1 H), 8.58 (m, 1 H), 8.19 (m, 1 H), 8.08 (m, 2H)

3. Radiosynthesis of 3-(4-Cyano-3-[$^{18}$F]fluorophenyl)-5-(trifluoromethyl)pyridine

From a cartridge in which [$^{18}$F] potassium fluoride produced in a cyclotron had been fixed, the radioactive isotope was eluted with a solution composed of 900 $\mu$L acetonitrile, 100 $\mu$L water, 20 mg Kryptofix®, and 30 $\mu$L 1 M $K_2CO_3$ solution. The eluted mixture was evaporated to dryness at 85 °C under an argon stream. The residue was then azeotropically dried two times each with 1 mL anhydrous acetonitrile under an argon stream. A solution of 5 mg of 3-(4-cyano-3-nitrophenyl)-5-(trifluoromethyl)pyridine was then added, and the mixture was heated at 160 °C for 20 minutes. After cooling to room temperature the solution was loaded directly onto HPLC (Kromasil® 100-10 C18, 5 mL/min $CH_3OH/H_2O$/triethylamine 60/40/0.1 vol/vol/vol). The radiochemical yield was 69%. A reaction scheme of this reaction is shown in Fig. 4.

4. Evaluation of inhibition of CYB11 B1 and CYP11 B2 by different inhibitors

For indirect evaluation of inhibition of CYB11 B1 and CYP11 B2 by different inhibitors, cells of the adrenocortical cell line NCI-H295 were cultured on 6-well plates by seeding 1x$10^6$ cells per well. NCI-H295 cells were obtained from ATCC (American Type Culture Collection) under ATCC number CRL-10296 via LGC Standards GmbH - Germany Office, Mercatorstr. 51, 46485 Wesel, Germany. NCI-H295 cells can also be obtained from Bioresource Collection and Research Centre (BCRC), 331, Shih-Pin Rd., Hsinchu 30062, Taiwan under BCRC number 60354. NCI-H295 cells produce cortisol as well as aldosterone. The cells contain the whole cascade of enzymes for producing these hormones. For determination of $IC_{50}$ values, the inhibitors were added to the culture medium at concentrations between 0.1 nM-10 $\mu$M and incubated for 48 hours. NCI-H295 cells treated in the same way but without inhibitor served as controls.

Y-1 is a continuous epithelial cell line derived from a murine adrenal adenocarcinoma. Y-1-cells are in the following designated "Y1-cells". They were obtained from CLS Cell Lines Service GmbH, Dr.Eckener-Str. 8, 69214 Eppelheim, Germany under order number 0477 MA. They can also be obtained from ATCC under ATCC number CCL-79. Y1-cells stably transfected with human CYP11 B1 gene (Y1-CYP11 B1 cells) and Y1-cells stably transfected with human CYP11 B2 gene (Y1-CYP11 B2 cells) were cultured on 6-well plates by seeding 5 x $10^5$ cells per well in 2 ml of culture medium. The enzyme reaction was started after 24 hours by addition of 1 ml culture medium containing either 1 $\mu$M RSS (21-Hydroxyprogesteron) or 1$\mu$M DOC (Deoxycorticosteronacetat) as substrate and the corresponding inhibitor at concentrations between 0.1 nM - 10 $\mu$M followed by incubation for 48 hours. RSS and DOC were dissolved in ethanol prior to addition to the culture medium. RSS and DOC are precursors of cortisol and aldosterone, respectively. These precursors are required because the cells do not contain the whole cascade of enzymes for synthesis of cortisol and aldosterone. Y1-Cyp11 B1 and Y1-Cyp11 B2 cells treated in the same way but without inhibitor served as controls.

Concentrations of cortisol and aldosterone in the supernatants of the cells were determined by commercially available radioimmunoassays (DPC Biermann, Bad Nauheim, Germany; IBL international, Hamburg, Germany). The

intraassay variance of both assays was <8%, the interassay variance <12%.

The $IC_{50}$ values are calculated according to the following general equation:

$$IC_{50} = \left(1 + \frac{[S]}{K_m}\right) \cdot K_i$$

S = Substrate concentration, $K_m$ = Michaelis-Menten constant, $K_i$ = Dissociation constant.

$IC_{50}$ values determined by use of Y1-CYP11 B1 cells are designated as "$IC_{50}$CYP11 B1" and $IC_{50}$ values determined by use of Y1-CYP11 B2 cells are designated as "$IC_{50}$CYP11 B2". A selectivity factor was calculated by the following formula:

$IC_{50}$CYP11 B1/$IC_{50}$CYP11 B2

The results of determinations of $IC_{50}$ values with various compounds according to the invention as inhibitors are as follows:

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 150 | 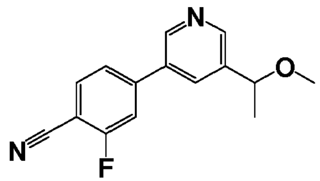 | 13.8 ± 4.46 (n=2) | 10.4 ± 1.9 | 1.3 | | | |
| 154 | 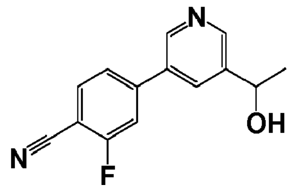 | 147 ± 121 (n=2) | 25.5 ± 2.4 (n=2) | 5.8 | | | |
| 155 | 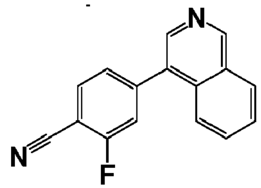 | 33.7 ± 27.2 | 37.0± 41.8 (n=2) | 0.9 | | | |
| 159 | 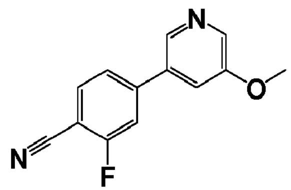 | 259 | 4.2 | 62 | 56 ± 36 | 2.3 ± 1 | 24 |
| 163 | 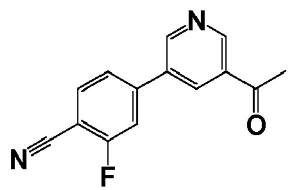 | 91.7 (n=1) | 164 (n=1) | 0.56 | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 180 | 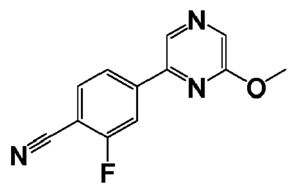 | 745± 203 | 8.7 ± 0.4 | 85.6 | 532 ± 275 | 5.7 ± 1.5 | 94.4 |
| 186 | 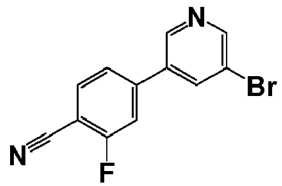 | 399 ± 521 | 7.2 ± 0.5 | 55.4 | 324 ± 206 | 11.0 ± 3.9 | 29.4 |
| 187 | 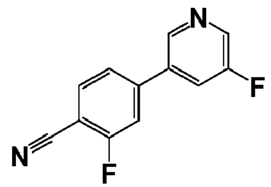 | 38.6 ±11.5 | 39.0 ± 11.4 | 0.9 | | | |
| 188 | 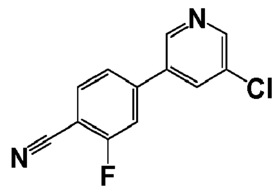 | 588 ± 327 | 9.6 ± 2.1 | 61.25 | 595 ± 362 | 10.6 ± 3.5 | 56.1 |
| 189 | 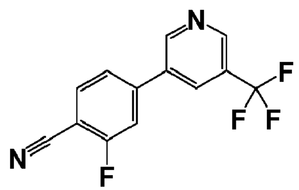 | 270 ± 60.3 | 10.1 ± 1.5 | 26.7 | 276 ± 142 | 6.7 ± 6.6 | 41.2 |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 191 | 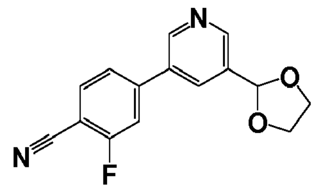 | 40.1 ± 16.2 | 0.98 ± 0.35 | 40.1 | 15.5 ± 8.1 | 1.1 ± 0.5 | 13.1 |
| 192 | 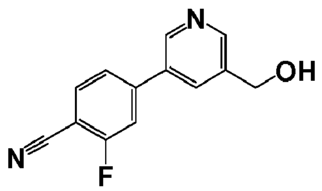 | 674± 346 | 12.0 ± 2.05 | 56.2 | 469± 228 | 11.4 ± 1.4 | 42.2 |
| 193 | 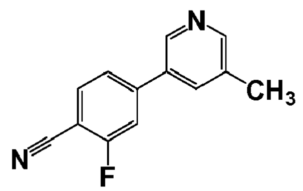 | 169 ± 74.4 | 2.9 | 58 | 47.8 ± 9.9 | 6.2 ± 2.4 | 7.7 |
| 194 | 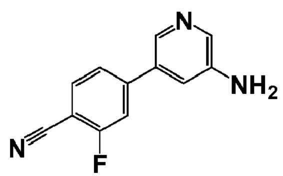 | 293 (n=1) | 47.6 (n=1) | 6.2 | | | |
| 195 | 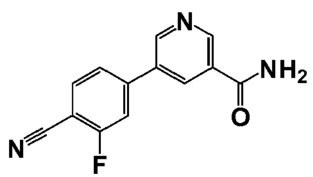 | >10,000 (n=1) | 582 (n=1) | / | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 196 | 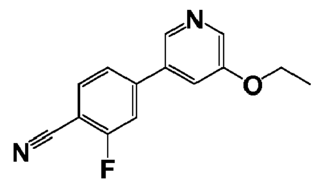 | 81.1 ± 21.8 | 2.6 ± 1.2 | 31.2 | 24.3 ± 22.7 | 1.9 ± 0.8 | 12.7 |
| 197 | 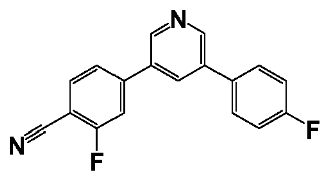 | 77.3 ± 30.3 | 1.6 ± 0.6 | 48.3 | 23.6 ± 18.8 | 1.1 ± 0.4 | 23.4 |
| 198 | 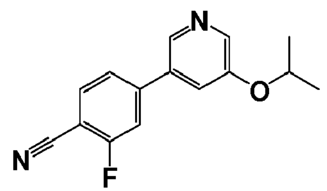 | 46.2 ± 27.8 (n=2) | 2.5 ± 2.3 | 18.5 | | | |
| 199 | 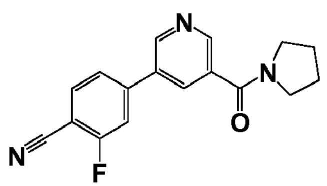 | 1666 (n=1) | 381 (n=1) | 4.3 | | | |
| 200 | 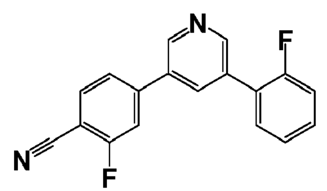 | 23.9 (n=1) | 1.7 (n=1) | 14 | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 203 | 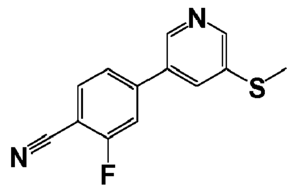 | 124 ± 39.5 | 4.6 ± 2.2 | 27.1 | 20.8 ± 14.0 | 3.8 ± 1.0 | 5.4 |
| 205 | 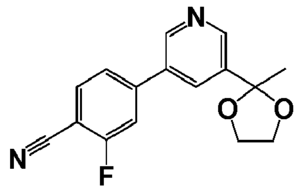 | 3.8 ± 2.2 | 0.3 ± 0.13 | 12.6 | | | |
| 206 | 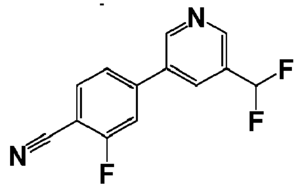 | 242 ± 134 (n=2) | 7.5 ± 4.1 | 32.2 | | | |
| 207 | 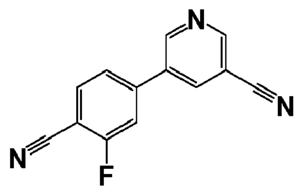 | 2100 ± 912 | 115 ± 60.1 | 18.7 | | | |
| 208 | 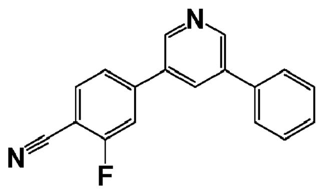 | 53.4 ± 34.7 (n=2) | 7.2 ± 7.5 | 7.4 | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 209 | 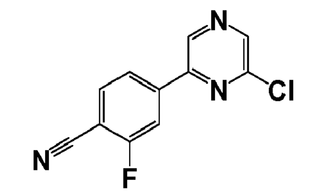 | >10,000 | 12.8 ± 4.7 | / | | | |
| 210 | 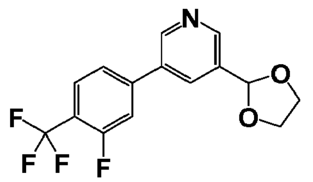 | 441 ± 17.5 | 3.8 | 116 | 157 ± 110 | 8.5 ± 5.2 | 27.8 |
| 214 | 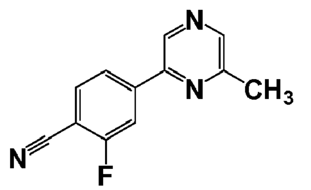 | 566± 374 (n=2) | 14.7± 5.3 (n=2) | 38.5 | | | |
| 218 | 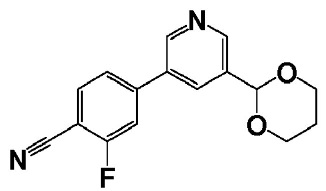 | 801 (n=1) | 13.6 (n=1) | 58.9 | | | |
| 219 | 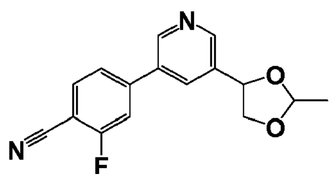 | 45.9 ± 15.3 | 0.94 ± 0.39 | 48.8 | | | |

(continued)

| Compound No. | Formula | Y1-Cells IC$_{50}$ CYP11 B1 [nM] | Y1-Cells IC$_{50}$ CYP11 B2 [nM] | Y1-Cells Selectivity factor | NCI-H295-Cells IC$_{50}$ CYP11 B1 [nM] | NCI-H295-Cells IC$_{50}$ CYP11 B2 [nM] | NCI-H295-Cells Selectivity factor |
|---|---|---|---|---|---|---|---|
| 221 | 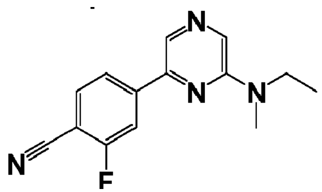 | 85.8 ± 90.8 | 2.2 ± 0.3 | 39 | | | |
| 222 | 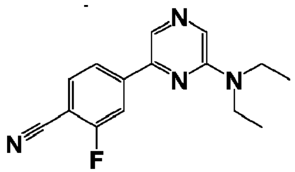 | 18.6 ± 4.03 | 0.75 ± 0.24 | 24.8 | | | |
| 223 | 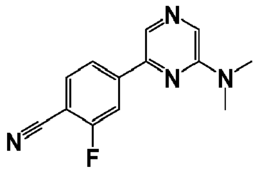 | 59.3 ± 11.0 | 0.94 ± 0.12 | 63.1 | | | |
| 224 | 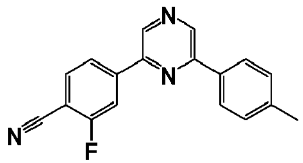 | 764 ± 147 | 11.8 ± 5.0 | 64.7 | | | |
| 225 | 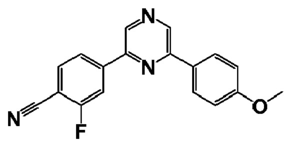 | 533 ± 154 | 12.5 ± 0.51 | 42.6 | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 226 | 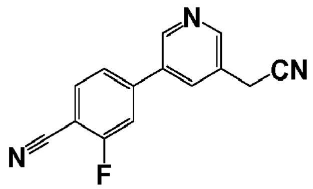 | 932 (n=1) | 50.8 (n=1) | 18.3 | | | |
| 227 | 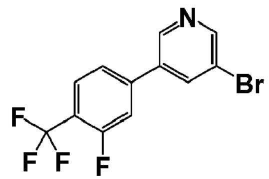 | 964 ± 843 | 30.6 ± 11.8 | 11.8 | | | |
| 228 | 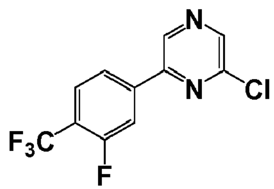 | 203 (n=1) | 81.0 (n=1) | 2.5 | | | |
| 229 | 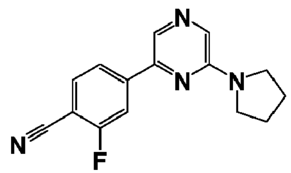 | 194 ± 35.5 | 1.5 ± 0.6 | 125.2 | | | |
| 230 | 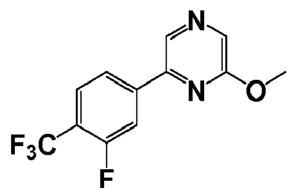 | >10,000 | 36.4 (n=1) | ca. 274 | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 231 | 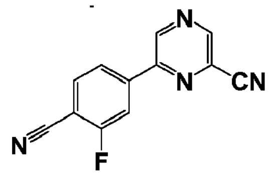 | 1500 ± 120 | 52.0 ± 19.4 | 28.8 | | | |
| 232 | 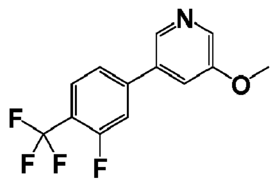 | 1448 ± 113 | 17 ± 0.7 | 85.2 | 258 ± 113 | 23.1 ± 6.3 | 11.2 |
| 233 | 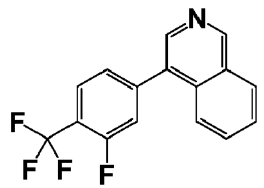 | 1184 ± 210 | 22.9 | 51 | 462 ± 294 | 16.6 ± 9.7 | 27.8 |
| 235 | 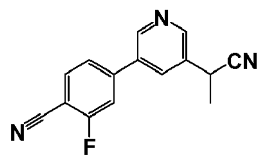 | 243 ± 45.2 | 4.6 ± 1.51 | 52.8 | | | |
| 236 | 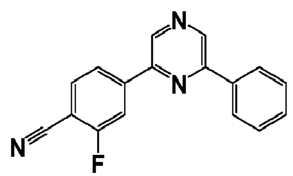 | 521 ± 109 | 11.2 ± 0.55 | 46.1 | | | |

(continued)

| Compound No. | Formula | Y1-Cells | | | NCI-H295-Cells | | |
|---|---|---|---|---|---|---|---|
| | | $IC_{50}$ CYP11 B1 [nM] | $IC_{50}$ CYP11 B2 [nM] | Selectivity factor | $IC_{50}$ CYP11 B1 [nM] | $IC_{50}$ CYP11 B2 [nM] | Selectivity factor |
| 237 | 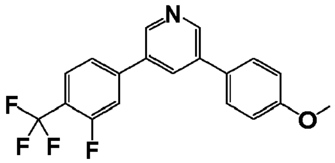 | 2130 ± 870 | 117 ± 137 | 18.2 | | | |
| 239 | 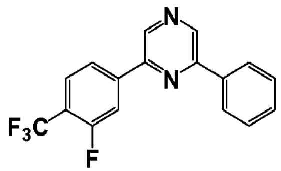 | >10,000 | 144 ± 31.0 | ca. 70 | | | |
| 240 | 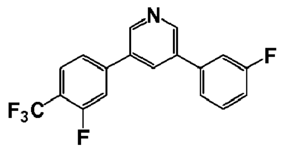 | >10,000 | 189 ± 84.9 | ca. 52 | | | |
| 241 | 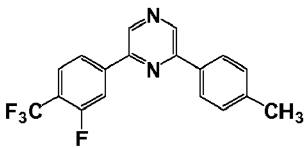 | 912 ± 781 | 623 ± 33.6 | 1.4 | | | |

5. *In vitro*-evaluation of different aldosterone synthase inhibitors

The binding of the $^{18}F$-labelled compounds no. 188, 189, and 191 to human cryosections of normal adrenal gland and conn-adenomas was studied. Results are shown in Fig. 5. The meaning of the abbreviations is as follows: nNN = normal adrenal gland, APA = aldosterone producing adenoma, CPA = cortisol producing adenoma, ACC = adrenocortical carcinoma, IMTO = $^{125}I$-Iodometomidate (an established tracer compound), A= 0.1 MBq of the radioactive compound + 10$\mu$M of the corresponding nonradioactive compound, B= 0.1 MBq of the radioactive compound.

Corresponding hematoxilin-eosin (HE) stained slices enlarged by a factor of 2.5 and 5, respectively are shown on the right side of Fig. 5. IMTO binds nonspecifically to CYP11 B1 and CYP11 B2 and serves as reference compound.

As shown in Fig. 5 best results with respect to a significant and specific binding were achieved with compound no. 191.

6. Determination of expression of target enzymes

The presence of target enzymes in every single slice used in cryosection experiments shown in Fig. 5 has been demonstrated by use of specific antibodies. Fig. 6 shows an CYP11 B1 and CYP11 B2 immunohistochemistry at paraffin tissue sections (upper row; A, B and C) and cryo tissue sections (lower row; D, E and F). A = CYP11 B2 normal adrenal gland 2.5x; B = CYP11 B1 normal adrenal gland 2.5x, C = Conn adenoma 2.5x, D = CYP11 B2 normal adrenal gland 2.5x, E = CYP11 B2 normal adrenal gland 10x, F = CYP11 B2 Conn adenoma 10x.

7. Characterization of nonradioactive CYP11 B2-inhibitors

Nonradioactive CYP11 B2 inhibitors according to the invention were characterized in *ex vivo* experiments using whole adrenal glands of humanized mice. The murine adrenal glands were coincubated with the respective inhibitors and secretion of aldosterone was measured as a function of time according to the following experimental setting: Immediately after removal of the adrenal gland, the organ was immersed in PBS, transferred to and immersed in 700 $\mu$l RPMI medium and incubated for 30 minutes at 37 °C. After this incubation time, 100 $\mu$l of the medium were removed and the basal concentration of aldosterone and corticosterone was determined. These basal concentrations are indicated as concentrations at the time 0 min and defined as 100% value. The enzyme expression was stimulated by adding ACTH (Adrenocorticotropes Hormone) to the immersed adrenal gland to a final concentration of 13.3 $\mu$g/l. At the same time, the inhibitor (10 $\mu$M) was added in a series of experiments followed by an incubation of the immersed adrenal gland at 37 °C. In a second series of experiments, only the corresponding volume medium, without inhibitor, was added. In both series of experiments, 100 $\mu$l of the supernatant of the immersed adrenal gland were removed in each case and concentrations of aldosterone or corticosterone in the supernatants were determined.

In these experiments, an inhibition of the aldosterone secretion in comparison to untreated adrenal glands was demonstrated for all investigated compounds. The results are shown in Figs. 7 to 9. The three-digit numbers in the legend on the upper right side of each of the figures designate the respective compounds according to the above table.

8. *In vivo* evaluation of influence of several substances and conditions and one compound according to the invention on aldosterone synthesis

An *in vivo* evaluation of the influence of several substances and conditions and one compound according to the invention on human aldosterone synthase was performed using mice expressing human aldosterone synthase.

Detection of inhibition of aldosterone synthesis is facilitated by a high aldosterone level. Therefore, the influence of a low-sodium-diet, of drinking water with high concentration of potassium for 24 h before start of the experiment, and of the age and sex of the mice was investigated in order to find out conditions resulting in a high aldosterone level. The result was that drinking water with a high concentration of potassium ions for 24 h before start of the experiment is able to increase the aldosterone level. The secretion of aldosterone varied with the age of the mice. Sex of the mice had no significant effect.

Compound no. 191 according to the above table showed inhibition of aldosterone synthase in *in vitro* experiments. The compound was tested *in vivo* and showed a potent inhibition of the secretion of aldosterone. Results are shown in Fig. 10. Fig. 10 shows the basal aldosterone level and the aldosterone levels one day after application of KCl in drinking water, 1 h after injection of iodometomidate (IMTO) i.v., and 1 h after injection of the CYP11 B2 inhibitor compound no 191 i.v..

## Claims

1. A compound having formula (I):

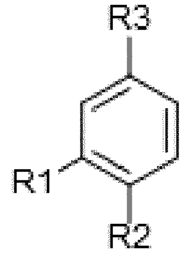

(I)

,

wherein R1 represents a radioactive isotope of an element which radioactive isotope is detectable by positron emission tomography (PET) or wherein R1 represents any nonradioactive isotope of said element, R2 represents -CN or $-CF_3$, wherein R3 represents

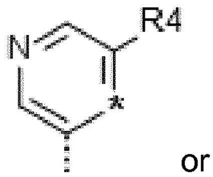 or 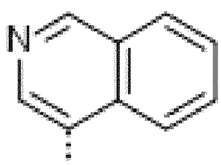 ,

wherein * represents C or N and R4 represents one of the moieties of a group consisting of alkyl, alkyl ether, alkyl nitrile, alkyl alcohol, alkyl halide, O-alkyl, S-alkyl, halogen, nitrile, trifluoromethyl, amino, aminoalkyl, aminodialkyl, phenyl, substituted phenyl, heterocyclic aromatic, substituted heterocyclic aromatic, dioxolanyl, methyldioxolanyl, and dioxanyl.

2. The compound of claim 1, wherein R1 is $^{18}F$.

3. The compound of claim 1 or 2, wherein R4 represents one of the following moieties: halogen, $-OCH_3$, $-CH_2OH$, $-CH_3$, $-N(CH_3)_2$, $-CH_2(CN)CH_3$, phenyl, substituted phenyl, heterocyclic aromatic and substituted heterocyclic aromatic.

4. The compound of any of the preceding claims, wherein the compound is

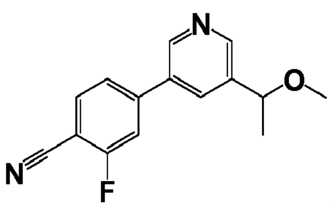 , 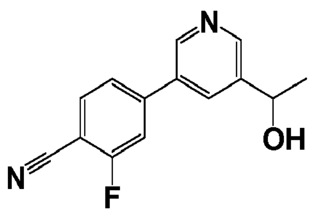 , 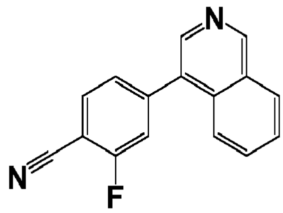 ,

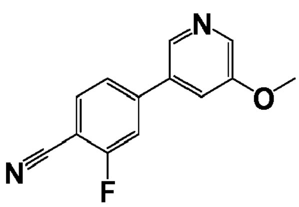 , 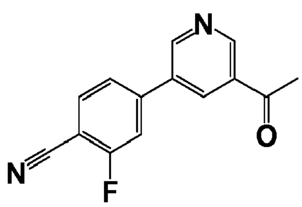 , 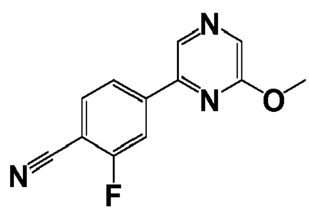 ,

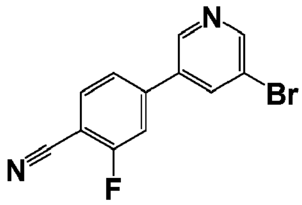, 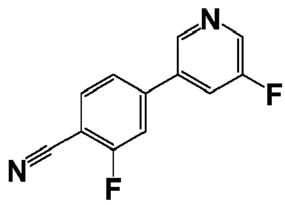, 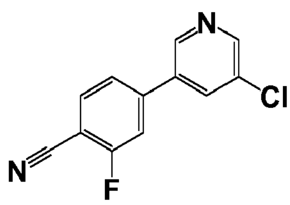,

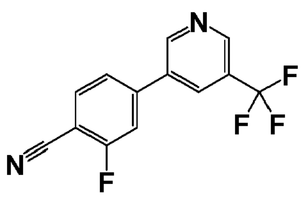, 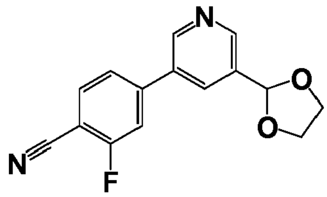, 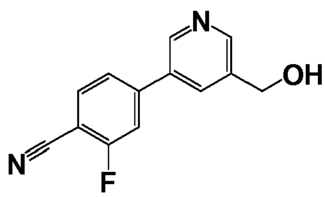,

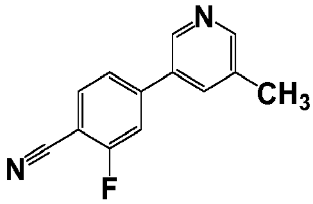, 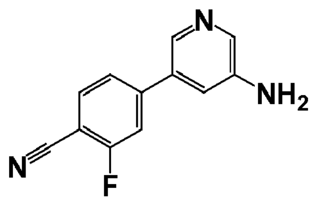, 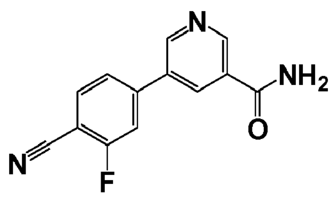,

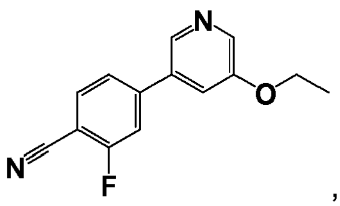, 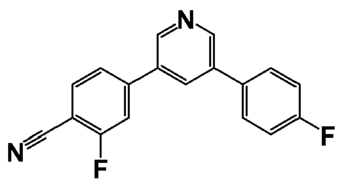, 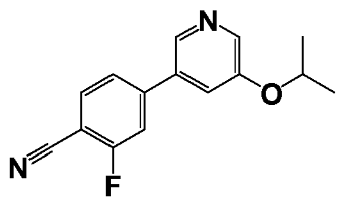,

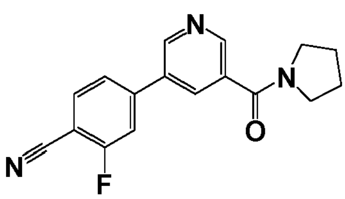, 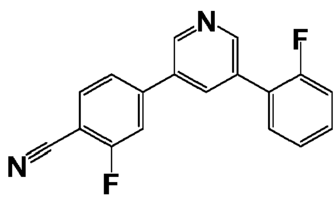, 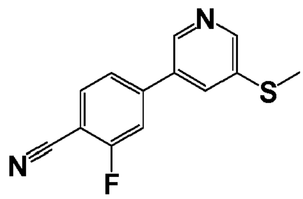,

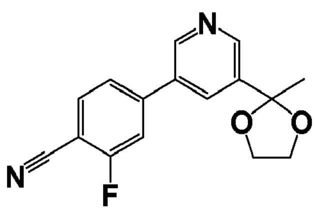, 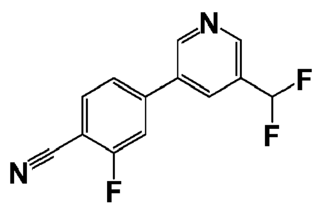, 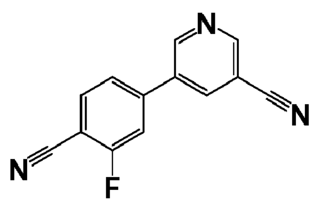,

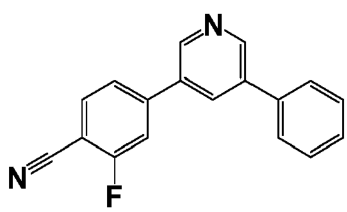, 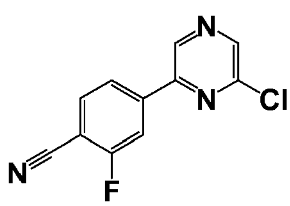, 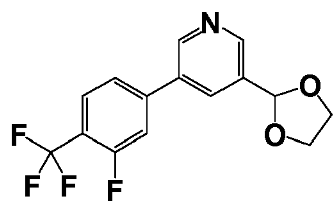,

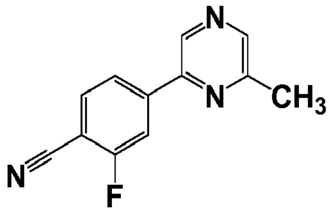,
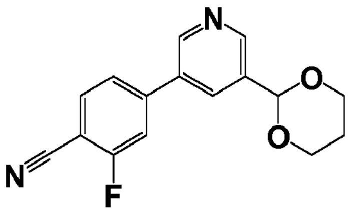,
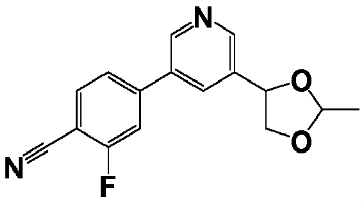,

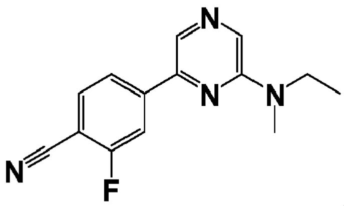,
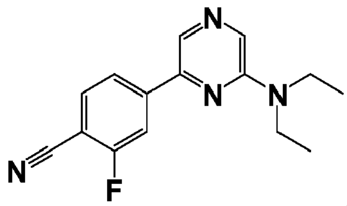,
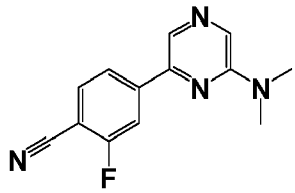,

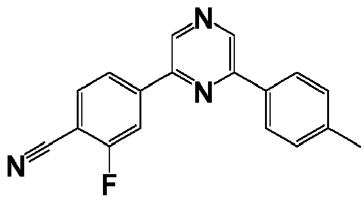,
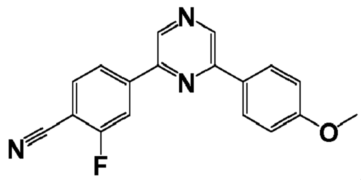,
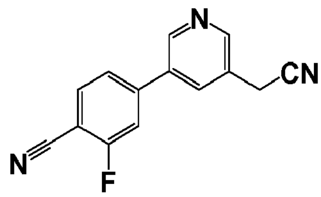,

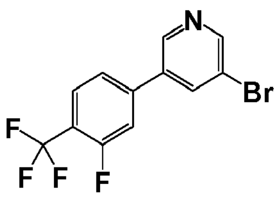,
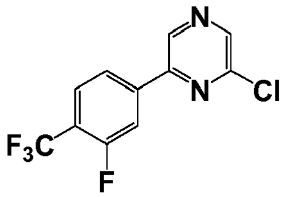,
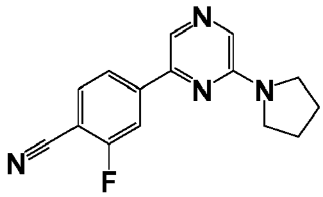,

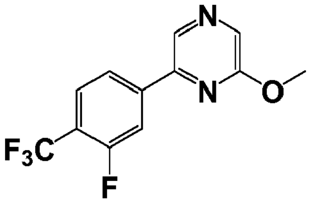,
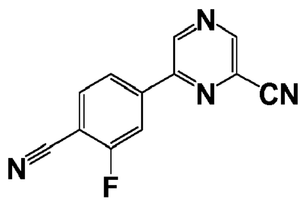,
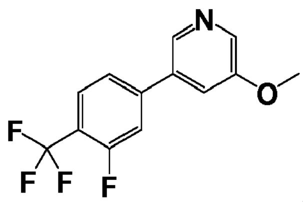,

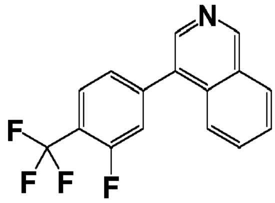,
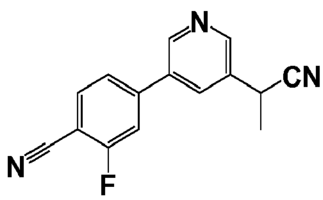,
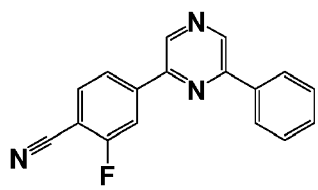,

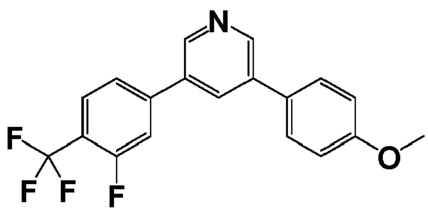,
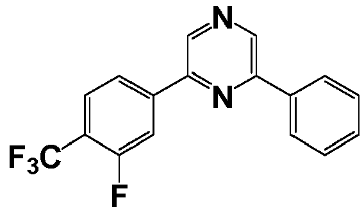,

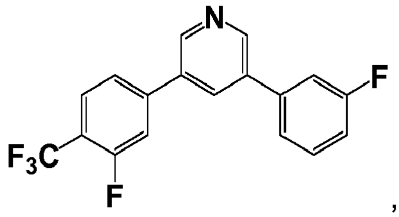

, or

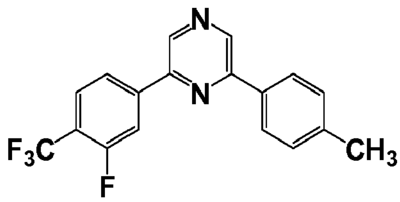

.

5. The compound of claim 4, wherein the F directly bound to the phenyl ring is $^{18}F$.

6. The compound of any of the preceding claims, wherein a specificity factor of the compound calculated as a relation of an $IC_{50}$ value of an enzymatic reaction of human CYP11 B1 to an $IC_{50}$ value of an enzymatic reaction of human CYP11 B2 is at least 10.

7. The compound of any of the preceding claims for use in an *in vivo* method of diagnosis.

8. The compound of claim 7 for use in an *in vivo* method of diagnosis, wherein the diagnosis is a functional diagnosis of a dysfunction of adrenal glands.

9. The compound of claim 8 for use in an *in vivo* method of diagnosis, wherein the dysfunction of adrenal glands is a unilateral form or a bilateral form of primary aldosteronism.

10. The compound of any of claims 7 to 9 for use in an *in vivo* method of diagnosis, wherein the method of diagnosis is positron emission tomography (PET).

11. The compound of claim 10 for use in an *in vivo* method of diagnosis, wherein the method of diagnosis comprises introducing the compound according to any of claims 1 to 6 into a mammal with adrenal glands and conducting PET in the region of the adrenal glands to obtain a functional image of the adrenal glands, wherein R1 represents the radioactive isotope.

12. Method for synthesizing the compound of any of claims 1 to 11, wherein a first precursor according to formula (II)

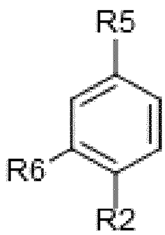

(II)

and a second precursor according to formula (III)

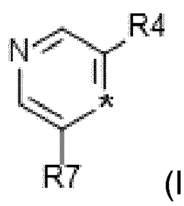

(III)

or a second precursor according to formula (IV)

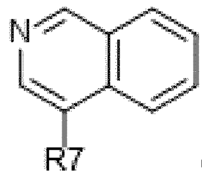

(IV)

are coupled with each other by a coupling reaction catalyzed by a catalyst, wherein R5 and R7 are moieties enabling the coupling reaction for coupling the first precursor with the second precursor, wherein R6 is R1 or a nitro group that is replaced by F or $^{18}F$ by a reaction with potassium fluoride or [$^{18}F$] potassium fluoride in the presence of a cryptand prior or after the coupling reaction.

**13.** Method according to claim 12, wherein the coupling reaction is a Suzuki reaction.

**14.** Method according to claim 13, wherein one of R5 and R7 is -$B(OH)_2$ and the other of R5 and R7 is a halogen moiety, in particular a bromine moiety, and/or the catalyst is tetrakis(triphenylphosphine)palladium(0).

**15.** Method according to any of claims 12 to 14, wherein the cryptand is 4,7,13,16,21,24-Hexaoxa-1,10-diazabicyclo[8.8.8]hexacosane ($C_{18}H_{36}N_2O_6$).

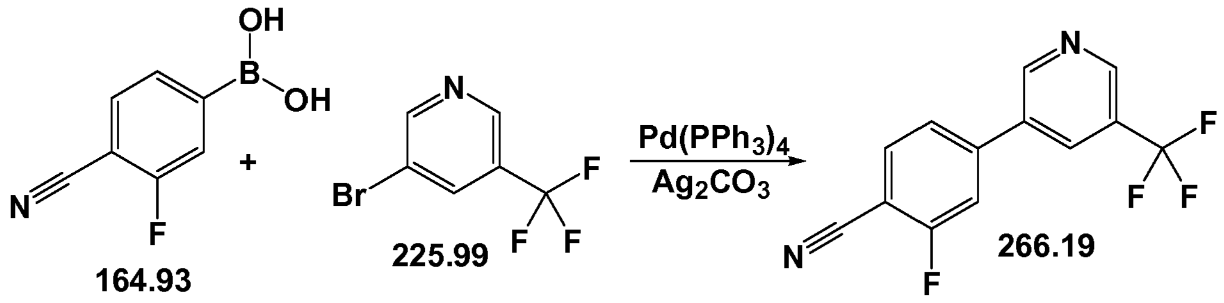


Fig. 1

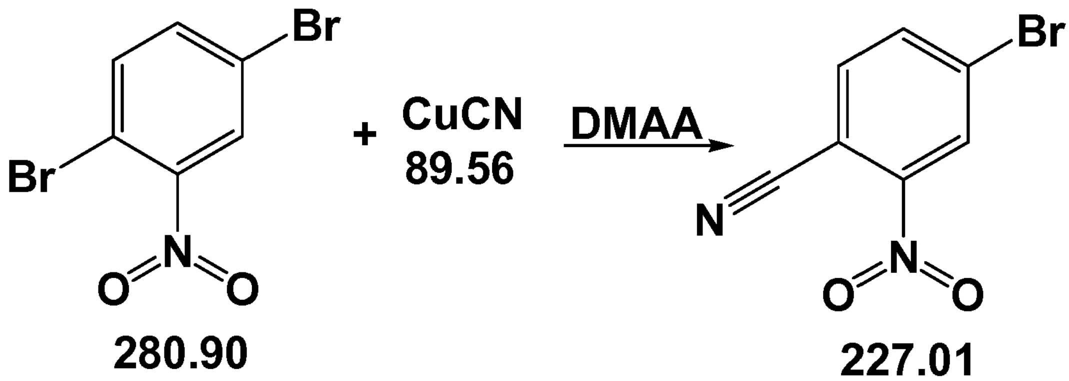


Fig. 2

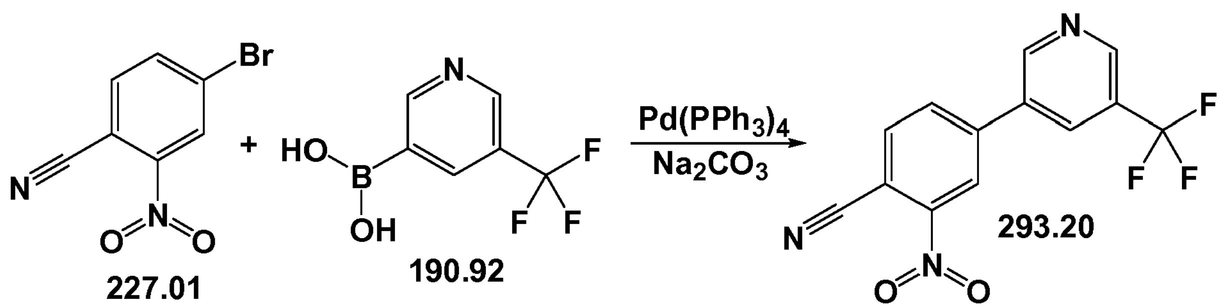


Fig. 3

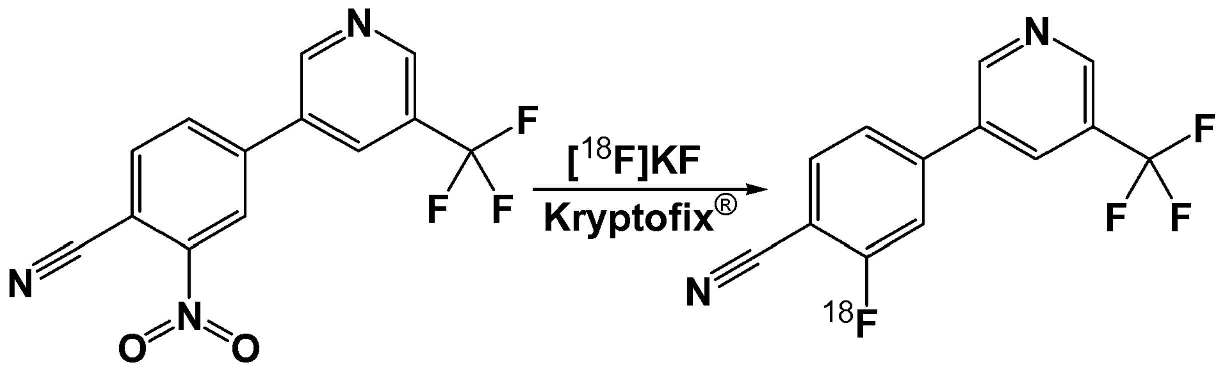
[18F]KF
Kryptofix®

Fig. 4

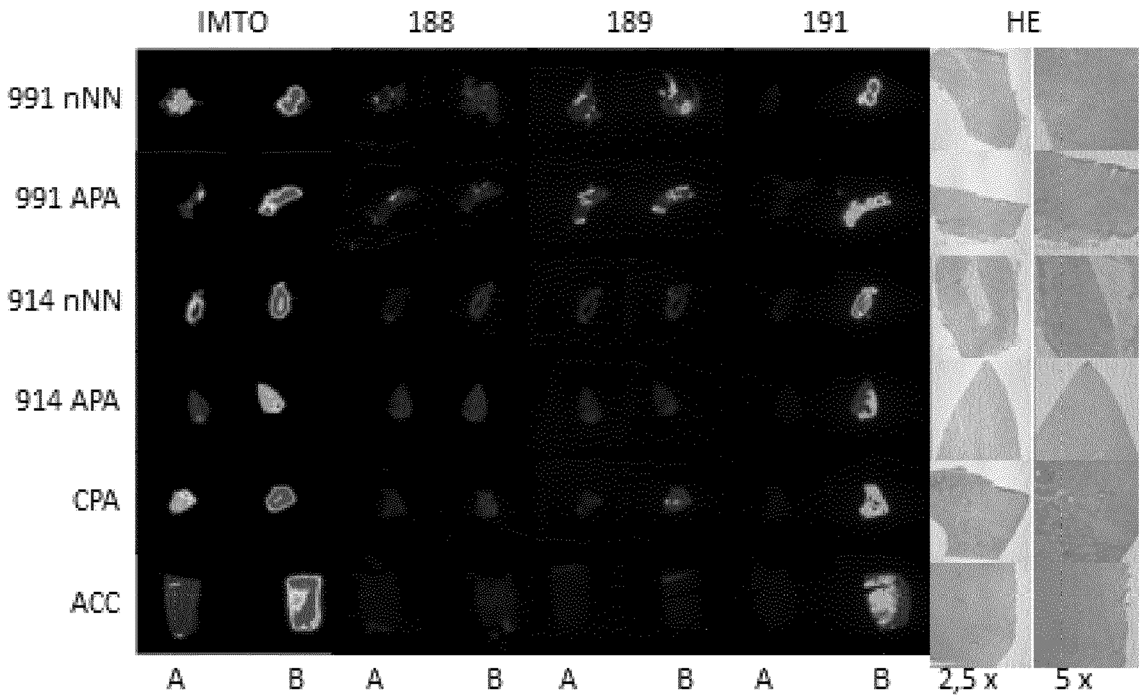
IMTO
188
189
191
HE
991 nNN
991 APA
914 nNN
914 APA
CPA
ACC
A
B
A
B
A
B
A
B
2,5 x
5 x

Fig. 5

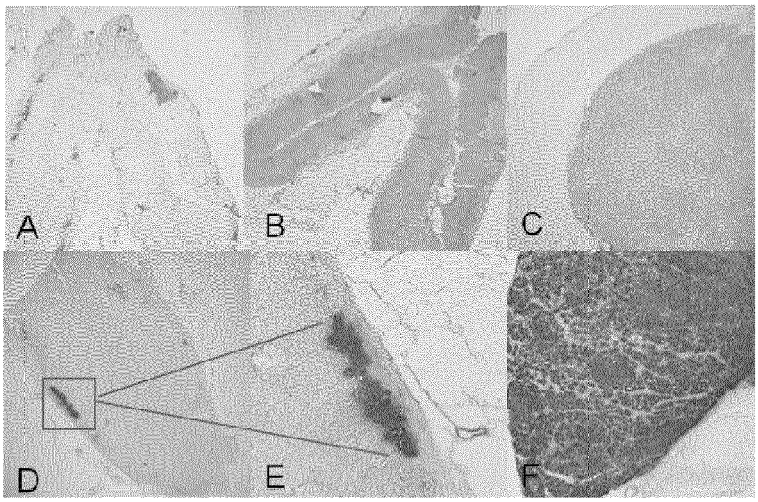
A
B
C
D
E
F

Fig. 6

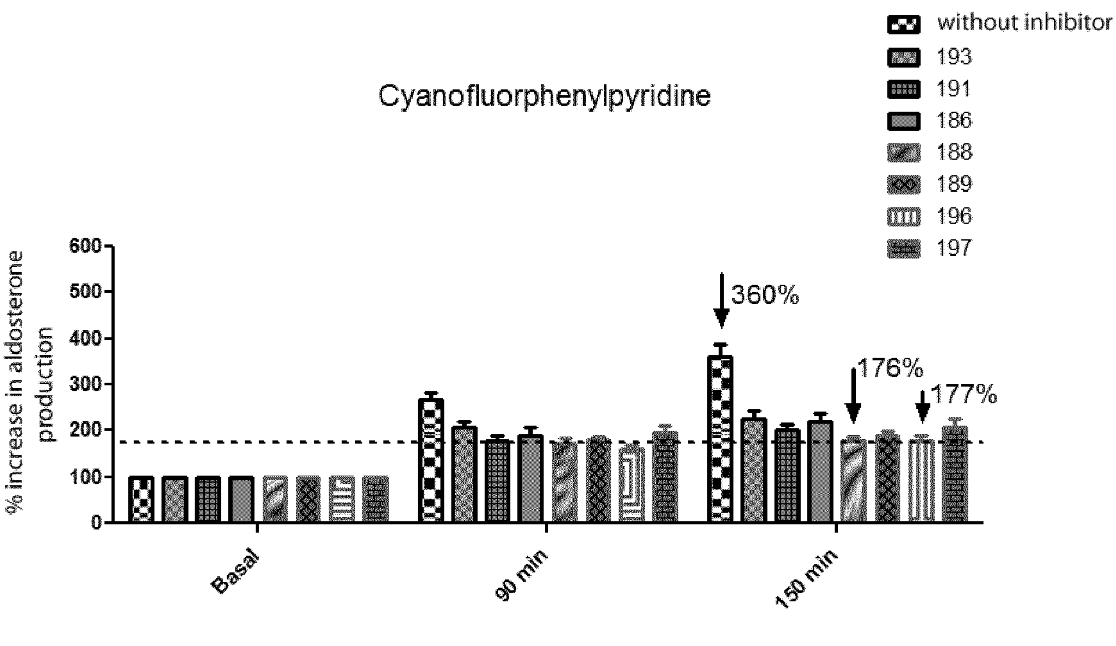
Cyanofluorphenylpyridine
without inhibitor
193
191
186
188
189
196
197
% increase in aldosterone production
600
500
400
300
200
100
0
360%
176%
177%
Basal
90 min
150 min

Fig. 7

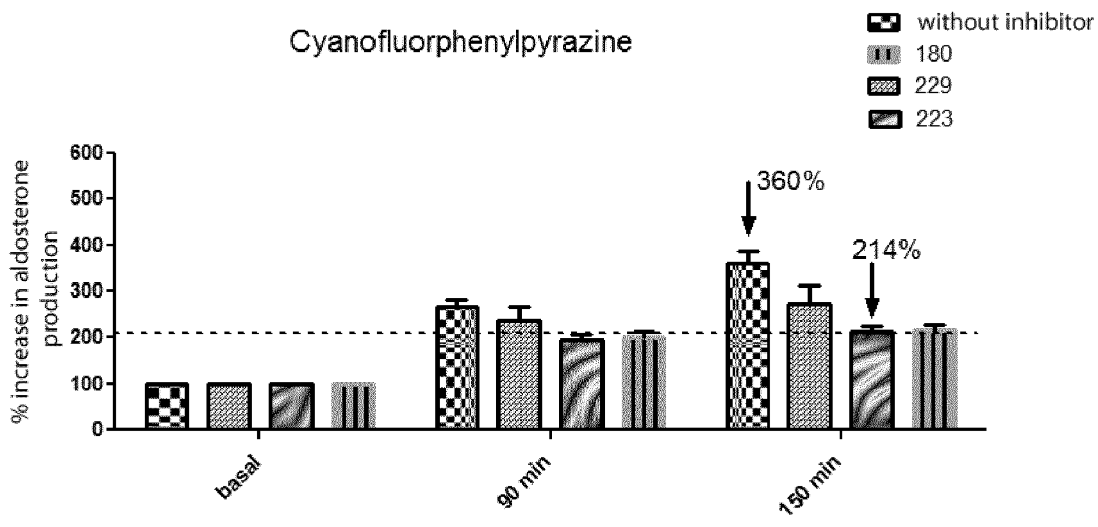
Cyanofluorphenylpyrazine
without inhibitor
180
229
223
% increase in aldosterone production
600
500
400
300
200
100
0
360%
214%
basal
90 min
150 min

Fig. 8

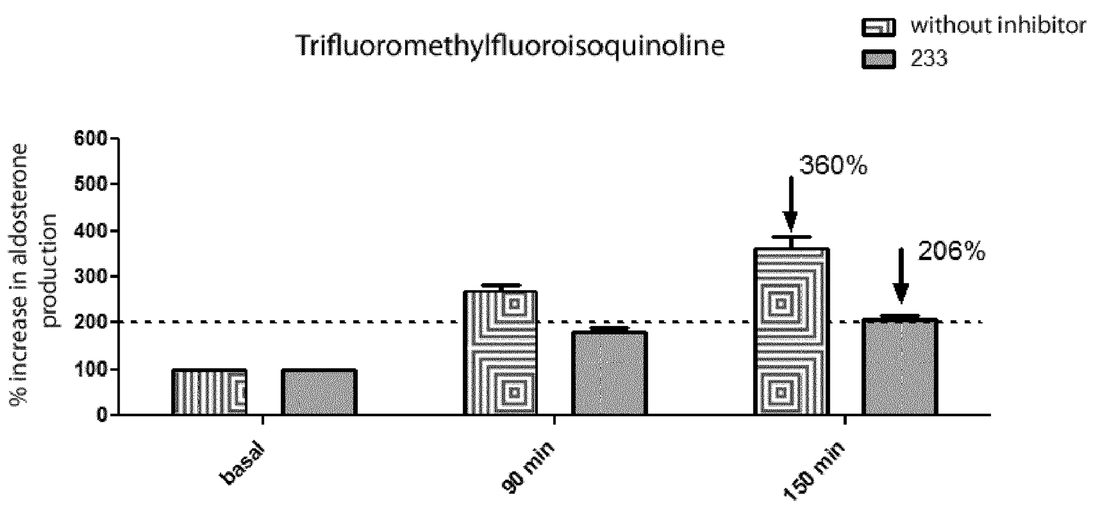
Trifluoromethylfluoroisoquinoline
without inhibitor
233
% increase in aldosterone production
600
500
400
300
200
100
0
360%
206%
basal
90 min
150 min

Fig. 9

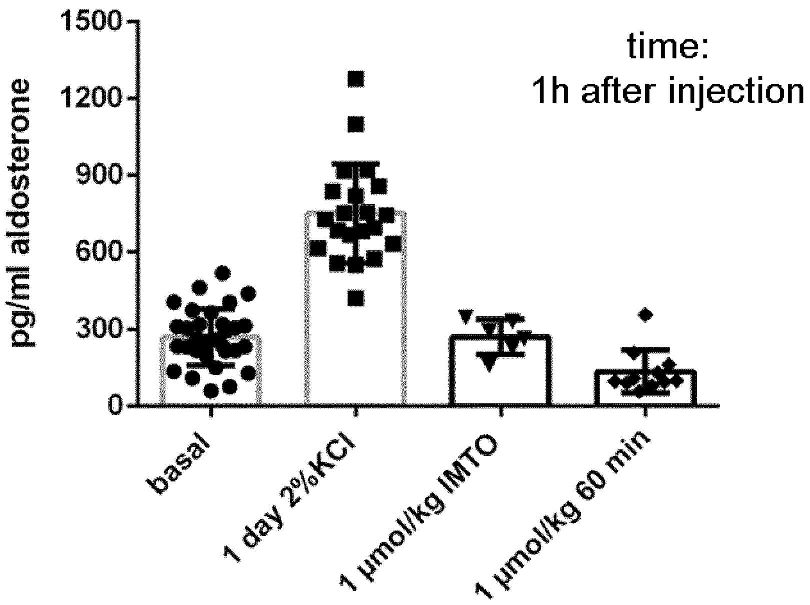
1500
1200
900
600
300
0
pg/ml aldosterone
time:
1h after injection
basal
1 day 2%KCl
1 µmol/kg IMTO
1 µmol/kg 60 min

Fig. 10

Europäisches Patentamt
European Patent Office
Office européen des brevets

# EUROPEAN SEARCH REPORT

Application Number
EP 17 15 4441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>A | WO 2010/130796 A1 (NOVARTIS AG [CH]; CHAMOIN SYLVIE [CH]; HU QI-YING [US]; PAPILLON JULIE)<br>18 November 2010 (2010-11-18)<br>* examples 17, step 2 *<br>* claims 1,15 *<br>----- | 1<br><br><br><br>2-15 | INV.<br>C07D213/73<br>A61K51/04<br>C07D213/74<br>C07D213/82<br>C07D213/84<br>C07D217/14<br>C07D405/04<br>C07D241/12<br>C07D241/16<br>C07D241/18<br>C07D241/20<br>C07D241/24<br>C07D213/57<br>C07D213/61<br>C07D213/65<br>C07D213/70<br>A61K31/44 |
| A | WO 2011/061168 A1 (NOVARTIS AG [CH]; ALLAN MARTIN [US]; CHAMOIN SYLVIE [CH]; HU QI-YING [] 26 May 2011 (2011-05-26)<br>* page 3, paragraph 2; claims 1,17-19 *<br>----- | 1-15 | |
| A | EP 2 394 668 A1 (UNIV WUERZBURG J MAXIMILIANS [DE])<br>14 December 2011 (2011-12-14)<br>* paragraph [0007] - paragraph [0010] *<br>* claims 1-4 *<br>----- | 1-15 | |
| | | | TECHNICAL FIELDS SEARCHED (IPC) |
| | | | C07D<br>A61K |

The present search report has been drawn up for all claims

1

| Place of search | Date of completion of the search | Examiner |
|---|---|---|
| Munich | 14 March 2017 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 15 4441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | | | A61K31/47<br>A61K31/4965 |
| | | | TECHNICAL FIELDS SEARCHED (IPC) |
| | | | |

The present search report has been drawn up for all claims

1

| Place of search | Date of completion of the search | Examiner |
|---|---|---|
| Munich | 14 March 2017 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 15 4441

This annex lists the patent family members relating to the patent documents cited in the above-mentioned European search report. The members are as contained in the European Patent Office EDP file on The European Patent Office is in no way liable for these particulars which are merely given for the purpose of information.

14-03-2017

| Patent document cited in search report | Publication date | Patent family member(s) | Publication date |
|---|---|---|---|
| WO 2010130796 A1 | 18-11-2010 | AR 076908 A1 | 20-07-2011 |
| | | AU 2010247414 A1 | 01-12-2011 |
| | | BR PI1010600 A2 | 15-03-2016 |
| | | CA 2761859 A1 | 18-11-2010 |
| | | CN 102803217 A | 28-11-2012 |
| | | CN 104892502 A | 09-09-2015 |
| | | CO 6470898 A2 | 29-06-2012 |
| | | CR 20110592 A | 04-01-2012 |
| | | CU 20110207 A7 | 15-02-2012 |
| | | DK 2429995 T3 | 22-04-2014 |
| | | EA 201101618 A1 | 29-06-2012 |
| | | EC SP11011526 A | 31-01-2012 |
| | | EP 2429995 A1 | 21-03-2012 |
| | | ES 2459468 T3 | 09-05-2014 |
| | | HK 1163666 A1 | 06-06-2014 |
| | | HR P20140371 T1 | 23-05-2014 |
| | | JP 5659224 B2 | 28-01-2015 |
| | | JP 2012526774 A | 01-11-2012 |
| | | KR 101338555 B1 | 09-12-2013 |
| | | MA 33358 B1 | 01-06-2012 |
| | | NZ 596302 A | 31-01-2014 |
| | | PE 04032012 A1 | 03-05-2012 |
| | | PT 2429995 E | 30-04-2014 |
| | | RS 53275 B | 29-08-2014 |
| | | SG 175925 A1 | 29-12-2011 |
| | | SI 2429995 T1 | 30-05-2014 |
| | | SV 2011004061 A | 04-01-2012 |
| | | TW 201100382 A | 01-01-2011 |
| | | US 2010292225 A1 | 18-11-2010 |
| | | US 2013035354 A1 | 07-02-2013 |
| | | US 2013296309 A1 | 07-11-2013 |
| | | UY 32640 A | 31-12-2010 |
| | | WO 2010130796 A1 | 18-11-2010 |
| | | ZA 201108155 B | 25-07-2012 |
| WO 2011061168 A1 | 26-05-2011 | CN 102712589 A | 03-10-2012 |
| | | EP 2501678 A1 | 26-09-2012 |
| | | EP 2993169 A1 | 09-03-2016 |
| | | ES 2551002 T3 | 13-11-2015 |
| | | JP 5654608 B2 | 14-01-2015 |
| | | JP 2013510896 A | 28-03-2013 |
| | | US 2012316195 A1 | 13-12-2012 |
| | | WO 2011061168 A1 | 26-05-2011 |
| EP 2394668 A1 | 14-12-2011 | EP 2394668 A1 | 14-12-2011 |
| | | EP 2575899 A1 | 10-04-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 17 15 4441

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2017

| Patent document cited in search report | Publication date | Patent family member(s) | Publication date |
|---|---|---|---|
| | | JP 5851493 B2 | 03-02-2016 |
| | | JP 2013534911 A | 09-09-2013 |
| | | US 2013089502 A1 | 11-04-2013 |
| | | WO 2011151411 A1 | 08-12-2011 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011151411 A1


### Non-patent literature cited in the description


- **ABE, T. et al.** *J. Clin. Endocrinol. Metab.,* March 2016, vol. 101 (3), 1008-15